# EUROPÄISCHE PATENTSCHRIFT

(11) **EP 1 803 425 B1**
(45) Veröffentlichungstag und Bekanntmachung des Hinweises auf die Patenterteilung: **03.12.2008**
(21) Anmeldenummer: 06026795.2
(22) Anmeldetag: 22.12.2006
(51) Int. Cl.: A61F 5/01, A61F 13/10

(54) **Orthopädisches Heilmittel**
Orthopaedic remedy
Remède orthopédique

(30) Priorität: 27.12.2005 DE 102005062477
(43) Veröffentlichungstag der Anmeldung: 04.07.2007
(73) Patentinhaber: Lohmann & Rauscher GmbH & Co. KG, 56567 Neuwied (DE)
(72) Erfinder:
(74) Vertreter: Flaccus, Rolf-Dieter

(56) Entgegenhaltungen:
- WO-A-86/04811
- WO-A-03/020185
- US-A- 4 628 918
- US-A- 5 865 775

## Beschreibung

Die vorliegende Erfindung betrifft orthopädische Hilfsmittel, die ein Grundelement mit einer inneren, körperseitigen und einer äußeren Oberfläche und mit mindestens einer Pelotte, und ein zumindest in einem Abschnitt elastisches Spannmittel zur Druckbeaufschlagung zumindest eines Bereichs der Pelotte aufweisen, wobei das Spannmittel ein Druckelement und einen dynamischen Zugindikator zur Einstellung des vom Druckelement ausgeübten Drucks auf die Pelotte enthält. Das erfindungsgemäße orthopädische Hilfsmittel kann z.B. eine Unterarmbandage zur Behandlung der Epicondylitis, wie sie häufig bei Golfspielern und Tennisspielern infolge monotoner Bewegungsabläufe und einer chronischen Reizung der Epicondylen auftritt, sein. Das Dokument WO-A-86/04811 stellt den nächster Stand der Technik dar.

Orthopädische Hilfsmittel wie Bandagen zur Therapie von überbeanspruchten und verletzten Gelenken und Muskelgruppen sind seit langem bekannt. Auch die Verwendung von eingearbeiteten Pelotten für eine gezielte Druck- und Massagewirkung auf Gelenke und Muskulatur zur Stabilisierung, Entlastung, Durchblutungsförderung und Schmerzlinderung ist bekannt. Hierzu zählen insbesondere Bandagen und Spangen zur Behandlung der Epicondylitis, des sogenannten Tennis- oder Golferellenbogens, jeweils in Abhängigkeit davon, welche Muskelgruppe des Unterarms betroffen ist.

Eine Epicondylitis ist oft Folge einer Überbeanspruchung durch monotone Wiederholung bestimmter Bewegungsabläufe bei körperlicher Tätigkeit. Durch eine Druckwirkung auf den an den Epicondylen wirkenden Muskelgruppen wird eine Senkung des Muskeltonus mit einer damit verbundenen fokussierten Entlastung der Sehnenansätze bewirkt, wodurch der der überbeanspruchte Bereich am Epicondylus entlastet und der Schmerz gelindert wird.

Da der durch eine Bandage, ein Spannelement oder eine Pelotte in einer Bandage oder durch eine Epicondylits-Spange ausgeübte Druck nicht so groß werden darf, daß die Durchblutung des Unterarms behindert wird, sind der Therapie zur Behandlung der Epicondylitis durch orthopädische Hilfsmittel hinsichtlich des maximal applizierbaren Druckes enge Grenzen gesetzt.

Zur Verbesserung der Druckwirkung, ohne die Durchblutung negativ zu beeinflussen, wurden daher verschiedene Ausführungen von Pelotten und/oder ein zusätzlicher Spanngurt über der Pelotte angewandt. So ist z.B. eine Epicondylitis-Bandage mit Spannriemen aus der europäischen Patentanmeldung EP 0 250 409 B1 oder aus der PCT-Anmeldung WO-A-86/04811 bekannt. Die Bandage besteht im wesentlichen aus einem Schlauchabschnitt aus elastischem Material, der sowohl einen Unterarm- als auch einen Oberarmabschnitt aufweist, wobei die Zugspannung in Umfangsrichtung über einen im wesentlichen in Umfangsrichtung verlaufenden Spannriemen mit Verschluß veränderbar ist. Bei dem elastischen Material des Schlauchabschnitts handelt es sich um ein Gestrick mit wärmedämmender Wirkung. An den Epicondylen entsprechenden Stellen sind im wesentlichen plattenförmige, hartelastische Einlagen vorhanden, wobei der Spannriemen so angeordnet ist, daß er diese zumindest teilweise überfaßt. Riemen und Einlagen sind dabei fest mit der elastischen Bandage verbunden, die einen Teil des Unter- und Oberarms sowie des Ellenbogens umschließt. Die Bandage ist in höchstem Maße rutschfest und soll die medizinisch richtige Lage der Einlagen gewährleisten.

Die im Stand der Technik beschriebenen Spannriemen sind im Verhältnis zu den verwendeten Pelotten relativ schmal, so daß der Druck nur lokal, meist im Zentrum der Pelotte ausgeübt werden kann. Eine Druckausübung auf den Rändern erfolgt nicht. Bei ungünstiger Materialwahl können sich die Ränder sogar hochbiegen und so diese Bereiche entlasten. Weiterhin wird der Druck unspezifisch auf die Pelotte ausgeübt, so daß ein gleichmäßiges Druckprofil nur schwer erreicht werden kann, wobei die Pelotte hierbei aber, wie in EP 0 250 409 B1 beschrieben, aus einem relativ festen Material bestehen muß, um den im engen Bereich des Gurtes erzeugten Druck gleichmä-ßig über ihre ganze Fläche abzuleiten.

Ein weiterer Nachteil des im Stand der Technik verwendeten Gurtes ist der, daß der Gurt, insbesondere bei körperlicher Betätigung, leicht verrutschen kann und so der Druck nicht mehr korrekt auf der richtigen Position der Pelotte ausgeübt wird, auch wenn ein Verrutschen der Bandage auf dem Arm durch die rutschfeste Ausführungsform verhindert wird.

Allerdings weisen die bisher verwendeten Vorrichtungen zur Druckbeaufschlagung auf eine Pelotte den Nachteil auf, daß die verwendeten Spanngurte aus einem wenig elastischem Material bestehen und kein Kontrollmittel für die Stärke des Zuges, respektive Drucks aufweisen, so daß leicht ein zu hoher Druck ausgeübt werden kann. Der ausgeübte Druck kann auch infolge eines geänderten Umfangs der zu behandelnden Stelle bei Beanspruchung der Muskulatur oder durch Ödeme ansteigen. Ebenso ist es möglich, den Gurt von vornherein zu fest anzuziehen, besonders da durch die Druckbelastung eine sofortige Schmerzlinderung erzielt wird, die den Anwender zu einer falschen Applikation verleitet. Durch diesen zu hohen oder gestiegenen Druck können dann die Durchblutung und auch der Heilungsprozess gestört werden.

Weiterhin ist der im Stand der Technik beschriebene Gurt fest mit der Bandage verbunden, so daß bei einer nichtsymmetrischen Ausführung diese immer nur am linken oder am rechten Arm verwendet werden kann. Im übrigen sind die im Stand der Technik beschriebenen Bandagen so gearbeitet, daß auch der Ellenbogen und ein großer Bereich des Oberarms vollständig umschlossen sind, was den Tragekomfort senkt, da selbst bei anatomisch gearbeiteten Bandagen Bewegungseinschränkungen und Faltenwurf im Gelenkbereich unvermeidbar sind, was bei langer Anwendungsdauer zu Irritationen und Reizungen führen kann.

Der vorliegenden Erfindung lag daher die Aufgabe zugrunde, ein orthopädisches Hilfsmittel, insbesondere zur Behandlung der Epicondylitis, zur Verfügung zu stellen, das die im Stand der Technik genannten Nachteile, wie die Störung der Durchblutung und schlechte Fixierung des Spannmittels auf der Pelotte, nicht aufweist, und das ferner einfach anzuwenden, günstig herzustellen und variabel einsetzbar ist. Zudem sollte die Pelotte in bestimmten Bereichen gezielt quer zum Muskel beaufschlagt werden können, um eine verbesserte therapeutische und vorbeugende Wirkung bei relativ geringem, durch den Anwender kontrolliertem Druck zu erreichen.

Die Aufgabe wird durch ein orthopädisches Hilfsmittel gemäß dem Hauptanspruch sowie durch die vorteilhaften Ausführungsformen der Unteransprüche gelöst.

Die orthopädischen Hilfsmittel der vorliegenden Erfindung enthalten dazu ein Grundelement mit mindestens einer Pelotte zur Druckbeaufschlagung und effektiven Massage der Muskulatur und der Sehnen. Außerdem enthalten sie ein Spannmittel zur zusätzlichen Druckbeaufschlagung zumindest eines Bereichs der Pelotte, wobei das Spannmittel einen Spannriemen, ein nicht elastisches Druckelement zur effektiven Druckübertragung und einen dynamischen Zugindikator zur Kontrolle des Zugs auf dem Spannmittel und somit zur Kontrolle des ausgeübten Drucks aufweist.

Die Vorteile der vorliegenden Erfindung liegen darin, daß durch die Verwendung des dynamischen Zugindikators ein übermäßiges Anziehen des Spannmittels verhindert wird und sich der auf die Pelotte ausgeübte Druck immer im therapeutisch optimalen Bereich befindet, ohne die Durchblutung des beaufschlagten Körperteils zu behindern. Durch die einfache Ablesbarkeit des Zugindikators ist der Anwender jederzeit in der Lage, den Zug bzw. Druck zu kontrollieren.

Durch die besondere Form des Druckelements ist weiterhin die gezielte Einwirkung auf bestimmte Bereiche der Pelotte möglich, so daß selbst bei geringer zusätzlicher Druckbeaufschlagung sowohl die Massagewirkung als auch die Entlastung des betroffenen Muskelsehnenapparates durch Senkung des Muskeltonusverbessert werden. Weiterhin kann durch die Form des Druckelements auf eine aufwendige strukturierte Ausarbeitung der Pelotten verzichtet werden, wodurch deren Herstellung vereinfacht wird.

Um die richtige Anordnung / Orientierung des Druckmittels auf dem Grundelement zu erleichtern, z.B. bei Anwendung am linken oder rechten Arm, kann auf dem Druckelement oder einem anderen Teil des Spannmittels ein charakteristisches Muster, Emblem, Schriftzug oder eine andere geeignete Markierung angebracht sein, die sich nur bei korrekter Ausrichtung des Druckelements bzw. Spannmittels auf dem Grundelement vom Anwender in korrekter Orientierung erkennen bzw. lesen lassen.

Das orthopädische Hilfsmittel der vorliegenden Erfindung weist ein Grundelement mit einer inneren, körperseitigen, und einer äußeren Oberfläche und mit mindestens einer Pelotte auf, die bevorzugt auf der inneren Oberfläche angeordnet ist. Weiterhin ist ein zumindest in einem Abschnitt elastisches Spannmittel zur Druckbeaufschlagung enthalten.

Das Grundelement kann z.B. eine Bandage oder auch eine Spange sein, in der die mindestens eine Pelotte derart angeordnet ist, daß sie bei medizinisch korrekter Applikation der Orthese die entsprechenden Muskeln und Sehnenansätze quer zur Muskulatur beaufschlagt und dem Anwender eine Schmerzlinderung oder anderen therapeutisch wünschenswerten Effekt verschafft.

Die Bandage kann dabei eine durchgehend gewebte Bandage oder eine in Längsrichtung vernähte Bandage sein. Die Bandage kann, bei Verwendung in der Nähe eines Gelenks, dieses umfassen oder aber auch unter oder über diesem enden. Die Bandage ist vorzugsweise mit einem elastischen Faden gewebt, wobei die Abschlüsse der Bandage weiter und/oder aus einem anderen Material gewebt sein können, um einen auslaufenden Druck an den Abschlüssen zu gewährleisten. Auch der Bandagenkörper kann aus unterschiedlichen Materialien gewebt sein. In einer bevorzugten Ausführungsform sind Bereiche im Beugebereich eines Gelenks mit einem nicht elastischen Faden gewebt, so daß diese besonders weich und hautfreundlich sind.

Wenn das Grundelement eine Spange darstellt, so kann deren dem Körper zugewandte Seite mit einer haptisch angenehmen Oberfläche wie einem Vlies oder Frotteestoff versehen sein. Der Träger der Spange kann aus einem flexiblen, leichten Material wie einem Metall oder Kunststoff gefertigt sein, wobei die Form des Trägers der Umfangsform des zu applizierenden Körperteils nachempfunden ist. In einer bevorzugten Ausführungsform wird der Träger der Spange durch das Druckelement des Spannmittels gebildet.

Die Pelotte ist aus einem Material hergestellt, das aus der Gruppe ausgewählt ist, die weichelastisch nicht kompressible Materialien, weichelastisch kompressible Materialien und nicht verformbare Materialien oder Kombinationen der zuvor genannten Materialien umfaßt. Beispiele für weichelastische nicht kompressible Materialien sind z.B. die Silikone und Kautschuke, während Silikon- und Polymerschäume zu den weichelastisch kompressiblen Materialien gehören. Bevorzugt wird die Pelotte aus Syntheselatex gefertigt, das weiterhin die Vorteile bietet, daß es preiswert, leicht und, im Gegensatz zu Naturlatex, antiallergen ist.

Die Pelotte hat bevorzugt eine Herz oder Nasenform, wobei auch jede andere geeignete Form zur Anwendung kommen kann, bei der bevorzugten Form aber verschiedene Muskelgruppen beaufschlagt werden können, so z.B. die Flexoren und Extensoren bei einer Epicondylitis. In einer ersten Ausführungsform weisen die Oberflächen der Pelotte keine erhabenen oder vertieften Bereiche auf. In einer weiteren, bevorzugten Ausführungsform besitzt die Pelotte bevorzugt auf der dem Arm abgewandten Seite quer zur Muskulatur verlaufende erhabene Bereiche, die im Falle einer Druckbeaufschlagung dieser Bereiche durch das Druckelement, diesen Druck an die darunterliegende Muskulatur weitergeben. Eines oder mehrere Bänder des Druckelements können auch zwischen den erhabenen Bereichen der dem Arm abgewandten Seite verlaufen, um das Druckelement so auf der Pelotte zu positionieren und zusätzlich zu fixieren. Alternativ kann die Pelotte auf der dem Muskel abgewandten Seite und/oder der dem Muskel zugewandten Seite Erhebungen aufweisen, die quer zum oder in Richtung des Muskels verlaufen oder frei orientierbar oder gekreuzt sind.

Die Pelotte kann in das Grundelement eingenäht oder in einer verschließbaren Tasche im Grundelement entnehmbar angeordnet sein, oder aber bewegbar und entfernbar auf der körperseitigen Oberfläche des Grundelements angeordnet sein, z.B. über ein Klettsystem, oder aber mit anderen geeigneten, dem Fachmann bekannten Mitteln am oder im Grundelement befestigt werden. Bevorzugte Positionen zur Anordnung der Pelotte können bei frei positionierbaren Pelotten z.B. durch farbige Flächen oder Umrisse auf der Innenseite des Grundelements gekennzeichnet sein.

Die Größe der Pelotten kann den verschiedenen Anwendungsbereichen oder an die Größen des orthopädischen Hilfsmittels, z.B. in Abhängigkeit des Armumfangs, angepaßt sein.

Ist mehr als eine Pelotte vorhanden, so wird eine symmetrische Anordnung derselben im Grundelement bevorzugt, insbesondere in Längsrichtung des Hilfsmittels, d.h. in Richtung der Achse des Armes im Fall eines orthopädischen Hilfsmittels zur Behandlung der Epicondylitis. Auf diese Weise wird die Anwendbarkeit des orthopädischen Hilfsmittels nicht auf eine Körperhälfte eingeschränkt. Bei entsprechender Indikation können die Pelotten aber auch nicht oder teilsymmetrisch angeordnet sein.
Wird mehr als eine Pelotte verwendet, können diese auch unterschiedlich geformt sein, aus unterschiedlichen Materialien bestehen und/oder unterschiedlich befestigt sein, je nach gewünschter Anwendung.

Die orthopädischen Hilfsmittel der vorliegenden Erfindung besitzen ferner ein Spannmittel, das im wesentlichen ein Druckelement und einen zumindest in einem Abschnitt elastischen Spannriemen mit einem Zugindikator enthält. Bevorzugt sind weiterhin ein erstes Befestigungsmittel zur Fixierung des Spannriemens unter Spannung, insbesondere ein Klettelement, und ein Umlenkelement enthalten. In einer weiteren bevorzugten Ausführung besitzt das Spannmittel ein zweites Befestigungsmittel zur Befestigung des Spannmittels auf dem Grundelement, wobei das zweite Befestigungsmittel vorzugsweise so ausgebildet ist, das das Spannmittel abnehmbar mit dem Grundelement verbunden ist.

Bevorzugte zweite Befestigungsmittel sind z.B. Klettsysteme oder Druckknöpfe, aber auch eine nichtabnehmbare Befestigung durch Vernähen, Verschweißen, Einspleißen und dergleichen ist denkbar. Das zweite Befestigungsmittel ist bevorzugt auf der Innenseite des Druckelements, das ist die dem Körper zugewandte Seite des Druckelements, angeordnet, wodurch das Druckelement zusätzlich korrekt auf der Pelotte positioniert wird. In einer alternativen Ausführungsform ist das Befestigungsmittel gegenüber dem Druckmittel auf der Innenseite des Spannmittels angeordnet.

In einer Ausführungsform mit einem Grundelement in Form einer Spange kann das Spannmittel auch integraler Bestandteil des Grundelements sein und ist fest mit diesem verbunden.

Der Spannriemen des Spannmittels umfaßt zumindest einen dehnbaren Abschnitt, auf dem der Zugindikator angeordnet ist. Ein Ende des Spannriemens ist fest mit dem Druckelement verbunden, während das freie Ende des Spannriemens stufenlos verstellbar mit dem ersten Befestigungsmittel auf dem Spannriemen selbst oder dem Grundelement zu befestigen ist.

Das Druckelement der vorliegenden Erfindung ist ein nicht oder nur sehr gering dehnbares (Dehnung bei Belastung < 5 %), mehrbändiges Druckelement. Das Druckelement weist bevorzugt mehrere Bänder auf. Die Breite des Druckelements im Verhältnis zur Breite der Pelotte kann frei gewählt werden, jedoch entspricht die Breite des Druckelements bevorzugt der Breite der zu beaufschlagenden Pelotte.

Das Druckelement kann aus jedem geeigneten Material hergestellt sein, das eine ausreichende Zugfestigkeit und Stabilität aufweist. Die Materialien können gering dehnungselastisches oder nicht dehnungselastische Gewebe und Kunststoffe, flexible biegeelastische Materialien und starre, nicht biegeelastische Materialien sein.
Bevorzugt wird das Druckelement aus einem thermoplastischen Polyurethan (TPU) gefertigt. Die Herstellung erfolgt bevorzugt in einem Spritzgußverfahren.

Das Druckelement besteht vorzugsweise aus einem nicht dehnbaren Material, wie z.B. einem Hartplastik, wobei die Form des Druckelements der Form der zu beaufschlagenden Stelle, z.B. des Unterarms, angepaßt ist und kann mehrere Bänder zur Druckbeaufschlagung aufweisen. Der Vorteil dieser Ausführung ist die gezielte Druckbeaufschlagung von einzelnen Bereichen der Pelotte, ohne die Möglichkeit des Verrutschens der Bänder relativ zueinander. Alternativ kann das Druckelement aber auch z.B. aus einem Nylon-Gewebe oder einem vergleichbaren Material gefertigt sein, das zwar leichter verrutschen kann, aber einen besseren Tragekomfort als die Hartplastikvariante bietet.

Die Länge des Druckelements beträgt zwischen 80 % bis 12 % des Umfangs des Anwendungsortes, bevorzugt zwischen zwischen 70 % bis 25 %, weiter bevorzugt zwischen 60 % bis 30, und besonders bevorzugt zwischen 50 bis 35 %.

Die Breite der einzelnen Bänder und Zwischenräume kann im Rahmen der dem Fachmann eingängigen Stabilitätsgrenzen der verwendeten Materialien frei gewählt werden, wobei die Anordnung und Breite der Bänder so gewählt wird, daß die druckausübenden Bänder auf den therapeutisch indizierten Bereichen der Pelotte zu liegen kommen. Weist die Pelotte, wie in einer bevorzugten Ausführung vorgesehen, erhabene Bereiche auf, so wird die Breite und Anordnung der Bänder so gewählt, daß diese bei Anordnung des Druckelements über der Pelotte mit deren erhabenen und/oder vertieften Bereiche korrespondiert, und daß die Bänder des Druckelements auf die unterliegenden Bereiche der Pelotte einen Druck ausüben.
Auf diese Weise kann ein maximaler Druck ausgeübt werden, ohne die Durchblutung des unterliegenden Gewebes zu beeinträchtigen.

Die Breite der Bänder beträgt zwischen 0,2 cm und 3,0 cm, bevorzugt zwischen 0,5 cm und 2,0 cm, weiter bevorzugt zwischen 1,0 cm und 1,7 cm, und besonders bevorzugt 1,5 cm. Die Breite der Zwischenräume beträgt zwischen 0,2 cm und 3 cm, bevorzugt zwischen 0,5 cm und 2 cm, weiter bevorzugt zwischen 0,7 cm und 1,5 cm, und besonders bevorzugt 1,0 cm, wobei die Breite der einzelnen Bänder und der einzelnen Zwischenräume unabhängig voneinander frei wählbar ist.

Die Anzahl der Bänder beträgt mindestens 1, kann aber bis zu 8 Bänder betragen, wobei 3-5 Bänder bevorzugt werden und 3 Bänder besonders bevorzugt, sind.

Die beiden äußersten Bänder des Druckelements können dabei oberarmseitung und unterarmseitig an den Rändern der Pelotte verlaufen, so daß ein verschieben des Druckelement aus seiner idealen Anordnung über der Pelotte durch die Bänder verhindert wird. Alternativ können, allein oder zusätzlich zur zuvor beschriebenen Ausführung, ein oder mehrere Bänder in Vertiefungen der Pelotte oder zwischen erhabenen Bereichen zur Erzielung desselben Effekts verlaufen.
Eine noch bessere Fixierung kann erreicht werden, wenn die Bänder, die die Position fixieren, tiefer im Druckelement liegen, d.h. kürzer sind, als die druckbeaufschlagenden Bänder.

In einer weiteren bevorzugten Ausführungsform verläuft das mittlere Band des Druckelements im ungespannten Zustand etwas über den äußeren Bändern. Diese Ausführungsform hat sich als vorteilhaft erwiesen, da bei Spannung des Spanngurtes und Biegung des Druckelements das mittlere Band dann in einer Ebene mit den äußeren Bändern zu liegen kommt.

In einer anderen Ausführungsform sind die Bänder des Druckelements in Richtung der Körperoberfläche vorgespannt/gewölbt, so daß auch bei minimaler zusätzlicher Spannung ein maximaler Druck aufgeübt werden kann.

In einer alternative Ausführungsform verlaufen die Bänder des Druckelements nicht quer zum Muskel, sondern mit dem Muskel. In diesem Fall kann dann, wie oben beschrieben, auch die Pelotte entsprechen angepaßt sein. Falls erforderlich ist auch jede andere Orientierung der Bänder relativ zum Muskel und in Abhängigkeit von der Gestalt der Pelotte möglich.

Unabhängig von der Art des Materials des Druckelements kann vorgesehen sein, daß die Bänder individuell oder gruppiert (z.B. paarweise, außenliegende oder innenliegende) spannbar im Druckelement angeordnet sind, so daß der therapeutische Druck auf einzelne Bereiche der Pelotte nach den therapeutischen Erfordernissen eingestellt werden kann. Die Bänder könnten bei dieser Ausführungsform z.B. an einem Ende fixiert und am freien Ende mit einer Vorrichtung, ähnlich der bei Kabelbindern, in der Länge eingestellt und befestigt werden. Ferner können sowohl Bänder als auch daraus resultierend die Zwischenräume asymmetrisch geformt sein, wenn dieses günstig für die zu erzielende Massagewirkung oder therapeutische Wirkung ist.

Das Druckelement kann außerdem an einem Ende ein Umlenkelement aufweisen, das bei Darstellung aus einem Kunststoff integraler Bestandteil des Druckelements ist. Weiterhin kann es zur Verbindung mit dem Spannriemen auf dem dem Umlenkelement gegenüberliegenden Ende eine Aufnahme für den Spannriemen aufweisen. Die Aufnahme kann ein zweites Umlenkelement sein, so daß sowohl das Druckelement als auch der Spannriemen symmetrisch aufgebaut sein können.

Alternativ ist aber auch eine Ausführungsform ohne Umlenkelement vorgesehen, so daß der Spannriemen z.B. direkt auf einem auf dem Spannmittel angeordnetem Flausch mit einem Hakenbandabschnitt befestigt wird.

In einer weiteren alternativen Ausführungsform ist der Spannriemen an seinem festen Ende direkt mit einem Druckelement mit Umlenkelement, das aus einem festen Kunststoff hergestellt ist, verbunden, so daß das Spannmittel ein einteiliges Element ist. Das Druckelement kann, wie schon erwähnt, aus dem Fachmann auf dem Gebiet bekannten Materialien hergestellt sein, wie z.B. Kunststoffen, Verbundstoffen, Metallen, Geweben oder anderen geeigneten Materialien oder Kombinationen dieser Materialien.

Der Spannriemen umfaßt zusätzlich einen Abschnitt aus einem elastischen Gewebematerial oder anderen geeigneten elastischen Materialien und einen nicht elastischen, nicht dehnbaren Abschnitt aus einem nicht dehnbaren Material, z.B. einem Nylongewebe. Der Spannriemen umfaßt neben dem Zugindikator, angeordnet auf dem elastischen Bereich, und dem nicht elastischen Bereich ein erstes Befestigungsmittel zur Befestigung des freien Endes des Spannriemens unter Spannung, wobei die Art des ersten Befestigungsmittels in Abhängigkeit von der Ausführungsform gewählt wird. Geeignete, dem Fachmann bekannte Befestigungsmittel sind z.B. Haken/Ösen, Klett, Riemen mit Schnallen und dergleichen.
Bei der bevorzugten Ausführung zur Befestigung des Spannriemens auf sich selbst nach Durchführung durch das Umlenkelement, ist das Befestigungsmittel ein Klettsystem, wobei Flausch und Haken auf einer Seite des Riemens angeordnet sind, so daß die Haken nach Umlenkung zum Arm gewandt sind und der Flausch dem Arm abgewandt ist. Diese Anordnung gewährleistet neben einer stufenlos variablen Befestigung des Spannriemens einen höheren Tragekomfort als bei inverser Anordnung.

Die Breite des elastischen Bereichs und des nicht elastischen Bereichs des Spannriemens sind bevorzugt gleich, können aber auch voneinander abweichen. Die Breite des Spannriemens liegt zwischen 1,5 cm und 20 cm, bevorzugt zwischen 2 cm und 15 cm, weiter bevorzugt zwischen 2,5 cm und 10 cm, und besonders bevorzugt zwischen 3,0 cm und 7 cm.

Über einen dynamischen Zugindikator (oder Druckindikator, wenn die mittelbare Wirkung des Riemens über die Pelotten auf den Arm zugrunde gelegt wird) läßt sich der durch das Spannmittel erzeugte Druck auf die Pelotte ablesen und der erforderliche Zug leicht einstellen. Sollten sich die Gegebenheiten ändern, z.B. bei Zuwachs des Muskelvolumens infolge körperlicher Anstrengung, kann mit diesem einfachen Hilfsmittel leicht überprüft werden, ob der Zug, und somit der über die Pelotte ausgeübte Druck, noch in den gewünschten Grenzen ist.

Der Zugindikator, besteht aus mindestens einer, bevorzugt mindestens zwei und besonders bevorzugt mindestens drei geometrischen Figuren, von denen mindestens eine Figur, im folgenden als Indikatorfigur bezeichnet, auf dem elastischen Bereich angeordnet ist. In Nachbarschaft zur Indikatorfigur kann auf einem nicht elastischen Bereich mindestens eine Referenzfigur angeordnet sein. Die Figuren sind dabei auf dem Spanngurt derart aufgedruckt, farbig eingewebt oder mit anderen geeigneten Mitteln kenntlich gemacht, daß sich die Form der Indikatorfigur mit der Dehnung infolge der Zugspannung auf dem dehnbaren Abschnitt ändert, während die Referenzfiguren, die auf einem nicht dehnbaren Bereich angeordnet sind, ihre Form nicht verändern.

Das Funktionsprinzip des dynamischen Zugindikators besteht darin, daß die Referenzfiguren eine von den Indikatorfiguren abweichende Form aufweisen, solange kein Zug ausgeübt wird. Wird ein Zug ausgeübt, so dehnt sich der elastische Bereich, auf dem die Indikatorfigur angeordnet ist, und die Figur verändert ihre Form mit der Dehnung. Entspricht die Form der Indikatorfigur der Form der Referenzfigur ist der Zug im gewünschten Bereich und der Riemen kann befestigt werden. Wird der Zug zu weit erhöht, so ergibt sich auch hier wieder eine Abweichung von der Referenzfigur durch eine zu starke Dehnung der Indikatorfigur und der Zug muß verringert werden.

Geeignete Beispiele für Figurenpaare aus Referenzfigur und ungedehnter Indikatorfigur sind z.B. Kreis/Ellipse, Quadrat/Rechteck, gleichseitiges Dreieck/Dreieck und dergleichen, wobei aber auch nicht geometrische Figuren oder Muster verwendet werden können, wenn diese geeignet sind. Bevorzugt weist der Zugindikator die Dreiecksform auf. Durch einen entsprechenden Aufdruck oder sonstig Aufbringung und Gestaltung der Figuren bei unterschiedlichen Dehnungen des elastischen Bereiches kann die gewünschte Zugkraft eingestellt werden, wobei eine Dehnung von 50% des elastischen Bereichs bei optimaler Zugkraft bevorzugt wird, da in diesem Fall eine maximale Toleranz sowohl gegen Dehnung als auch Entspannung gegeben ist.
Bei einer eindeutigen Zuordnung oder Beschreibung der Soll-Form oder Zielform der Indikatorfigur (z.B. Kreis, Quadrat, gleichseitiges Dreieck) kann auch vollständig auf Referenzfiguren und somit auf einen nicht dehnbaren Abschnitt im Spannriemen verzichtet werden.

In einer weiteren Ausführungsform ist nicht die Form, sondern die Farbe des Indikators entscheidend. So kann das Referenzfeld z.B. eine hellere Färbung aufweisen als das Indikatorfeld im Ausgangszustand. Durch Dehnung wird die Farbdichte des Referenzfeldes dann geringer und die Farbe heller. Bei Farbgleichheit ist ein optimaler Zug erreicht.

Die orthopädischen Hilfsmittel der vorliegenden Erfindung zeichnen sich dadurch aus, daß sie zur Behandlung von Epicondylitis und verwandten Erkrankungen geeignet sind, ohne einen zu großen Druck auf die beaufschlagten Körperareale auszuüben. Die Orthesen der vorliegenden Erfindung besitzen ein Grundelement, das eine Bandage oder auch eine Spange sein kann, das mindestens eine Pelotte aufweist, und ein Spannmittel umfassend ein Druckelement, einen Spannriemen mit Befestigungsmittel und einen Zugindikator.
In einer bevorzugten Ausführungsform ist das orthopädische Hilfsmittel eine Bandage mit einem lösbar befestigen Spannmittel, wobei diese Kombination den Vorteil bietet, daß das Grundelement, in diesem Fall eine Bandage mit eingearbeiteter Pelotte, bei normaler Belastung ohne das Spannmittel getragen werden kann, was den Tragekomfort für den Anwender erhöht. Erst bei einer Belastung der betroffenen Muskeln Sehnen und Gelenke wird zusätzlich das Spannmittel auf der Bandage befestigt und eingesetzt, so daß das orthopädische Hilfsmittel seine volle Wirksamkeit entfaltet.

In einer weiteren bevorzugten Ausführungsform umschließt die Bandage das Gelenk nicht, sondern endet unterhalb des Ellenbogens, so daß die Bewegungsfreiheit nicht eingeschränkt wird und Reizungen im Gelenkbereich vermieden werden.

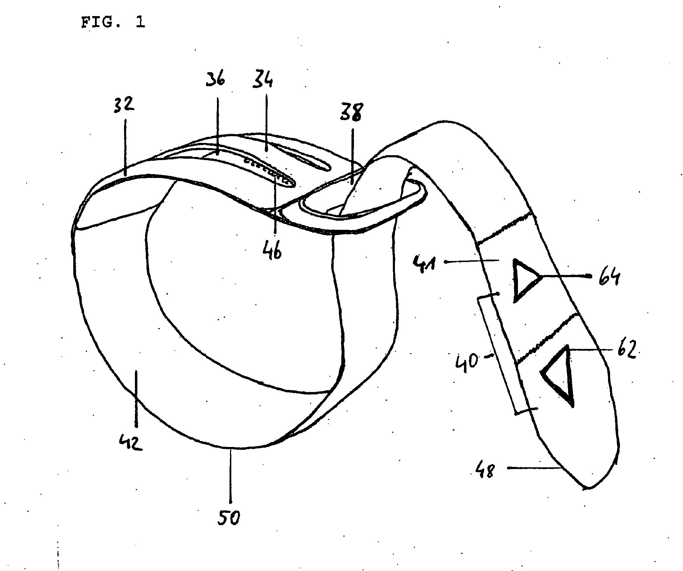

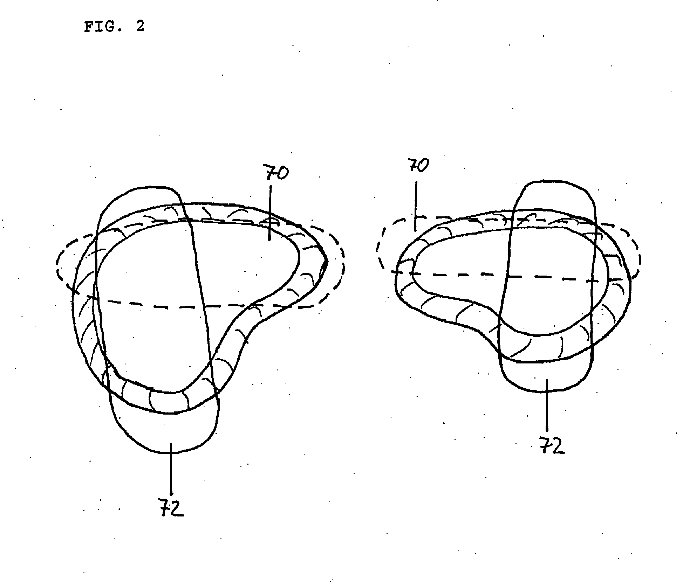

Die Erfindung wird durch die Figuren 1 bis 4 und die nachfolgenden Beispiele noch weiter verdeutlicht, wobei die Figuren im folgenden zeigen:
Figur 1 zeigt ein Spannmittel gemäß Anspruch 1 der vorliegenden Erfindung mit Druckelement, Zugindikator und Spannriemen.
Figur 2 zeigt schematisch zwei verschiedene Pelotten in Herz oder Nasenform in denen die Bereiche angedeutet sind, die auf die Extensoren und Flexoren des Unterarms einwirken.
Figur 3 zeigt die Ausführungsform des erfindungsgemäßen orthopädischen Hilfsmittels als Bandage mit Druckindikator.
Figur 4 zeigt die Ausführungsform des erfindungsgemäßen orthopädischen Hilfsmittels als Spange.

### Beispiel 1:

In einer bevorzugten Ausführungsform ist das orthopädische Hilfsmittel (10) eine Bandage (60) zur Behandlung oder Vorbeugung von Epicondylitis ist. Das Grundelement (12) des Hilfsmittels ist eine Unterarmbandage mit zwei symmetrisch angeordneten, oberflächenstrukturierten Pelotten (20) aus einem weichelastischem Material, z.B. Silikion oder Syntheselatex, die auf die an dem äußeren und inneren Epicondylus endenden Muskeln und Sehnen einwirken. Die Bandage (60) besteht aus einem elastischem Gewebe, in das die Pelotten (20), die zwei quer zum Arm verlaufende erhabene Bereiche (22) aufweisen, fest eingenäht sind, wobei das armseitige Gewebe weich ist, so daß es hautfreundlich erscheint. Die Bandage (60) ist mit einer Naht, die in Armrichtung an der Außenseite des Arms verläuft, zu einem Schlauch zusammengenäht.

Die Bandage (60) überkappt oder umfaßt nicht den Ellenbogen oder den Oberarm, sondern endet an der Ellenbeuge, wobei im Bereich der Ellenbeuge ein Druckentlastungsbereich (18) eingearbeitet ist, um Einschränkungen, Irritationen und Reizungen in der Ellenbeuge durch die Bandage (60) beim Beugen des Arm zu verhindern. Der Druckentlastungsbereich (18) ist ein lockerer gewebtes, weicheres Gewebe, das in Form eines Kreisausschnitts in den Bandagenbereich läuft und Bestandteil des oberarmseitig verlaufenden, ebenfalls lockerer gewebten Abschlusses ist. Das handseitige Ende der Bandage (60) weist ebenfalls einen weiter gewebten Abschluß auf.

Das erfindungsgemäße Spannmittel (30) besteht aus einem Druckelement (32) aus einem anatomisch geformten Kunststoff, z.B. thermoplastisches Polyurethan (TPU), mit Umlenkelement (38) und einem Spannriemen (42) mit Zugindikator (40) und Klettsystem (44) zur stufenlos variablen Befestigung des Spannriemens (42). Das Ende des Spannriemens (42) weist dabei einen Hakenbandabschnitt (48) auf, während der der übrige Bereich des Spannriemens (42), außer dem elastischen Bereich, mit einem Flausch (50) bedeckt ist.

Das Druckelement (32) besitzt drei symmetrisch angeordnete Bänder (34) und zwei Zwischenräume (36), wobei die äußeren Bänder (34) über den erhabenen Bereichen (22) der Pelotten (20) zu liegen kommen, während das innere Band zwischen ihnen verläuft. Die Länge des Druckelements (32) ist so gewählt, daß es nur über eine der beiden Pelotten (20) fassen kann, während die andere lediglich vom Spannriemen (42) beaufschlagt wird.

Das Druckelement (32) enthält an einem Ende ein Umlenkelement (38), während an seinem anderen Ende der Spannriemen (42) fest mit dem Material des Druckelements (32) verbunden ist.

Das Spannmittel (30) ist armseitig in einem Bereich mit einem Hakenband (46) versehen, das auf einem korrespondierenden Flausch auf der Bandage (60) (Grundelement (12)) befestigt werden kann, wobei der Bereich bevorzugt dem Druckelement (32) gegenüber liegt.

Der Zugindikator (40) des Spannriemens (42) besitzt auf beiden Seiten des elastischen Bereichs eine Referenzfigur (62) in Form eines gleichseitigen Dreiecks, während die Form der Indikatorfigur (64) ebenfalls ein Dreieck (nicht gleichseitig im ungedehnten Zustand) ist.
Das Spannmittel (30) besitzt eine Breite von ca. 4 cm, das Druckelement (32) von ca. 6 cm, wobei die Bänder (34) eine Breite von ca. 1,5 cm und die Zwischenräume (36) von ca. 1 cm aufweisen, und wobei die Länge des Druckelements (32) ca. 11 cm beträgt.

### Beispiel 2:

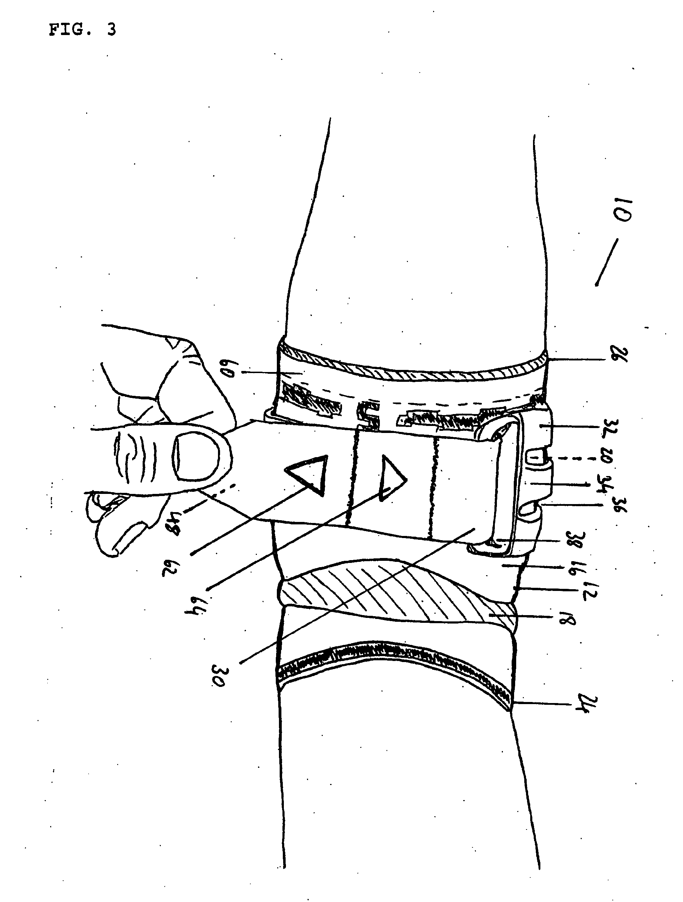

In einer weiteren Ausführungsform, dargestellt in Figur 3, ist das orthopädisches Hilfsmittel (10) eine Bandage (60) zur Behandlung oder Vorbeugung von Epicondylitis. Das Grundelement (12) des Hilfsmittels ist eine Unterarmbandage mit zwei symmetrisch angeordneten Pelotten (20) aus einem weichelastischem Material, z.B. Silikon oder Syntheselatex, die auf die an dem äußeren und inneren Epicondylus endenden Muskeln und Sehnen einwirken. Die Bandage (60) besteht aus einem elastischem Gewebe, in das die Pelotten, die keine erhabenen Bereiche (22) aufweisen, fest eingenäht sind, wobei das armseitige Gewebe weich, hautfreundlich und angenehm zu tragen ist. Die Bandage (60) ist mit einer Naht, die in Armrichtung an der Außenseite des Arms verläuft, zu einem Schlauch zusammengenäht.

Die Bandage (60) überkappt oder umfaßt den Ellenbogen im Beugebereich nur um 1,5 cm - 3 cm, während sie auf der Seite des Ellenbogens den Oberarm ca. 5 cm umfaßt. Im Bereich der Ellenbeuge ist in die Bandage (60) ein Druckentlastungsbereich (18) eingearbeitet, um Einschränkungen, Irritationen und Reizungen in der Ellenbeuge durch die Bandage (60) beim Beugen des Arm zu verhindern. Der Druckentlastungsbereich (18) ist ein lockerer gewebtes, weicheres Gewebe aus einem nicht elastischen Faden, das in Form eines Kreisausschnitts in den Bandagenbereich läuft. Das oberarmseitige und handseitige Ende der Bandage (60) weisen ebenfalls einen weiter gewebten Abschluß auf.

Das erfindungsgemäße Spannmittel (30) besteht aus einem Druckelement (32) aus einem anatomisch geformten, flexiblen Kunststoff, z.B. TPU, mit Umlenkelement (38) und einem Spannriemen (42) mit Zugindikator (40) und Klettsystem (44) zur stufenlos variablen Befestigung des Spannriemens (42). Das Ende des Spannriemens (42) weist dabei einen Hakenbandabschnitt (48) auf, während der übrige Bereich des Spannriemens (42), außer dem elastischen Bereich, mit einem Flausch (50) bedeckt ist.

Das Druckelement (32) besitzt drei symmetrisch angeordnete Bänder (34) und zwei Zwischenräume (36). Die Länge des Druckelements (32) ist so gewählt, daß es nur über eine der beiden Pelotten (20) fassen kann, während die andere lediglich vom Spannriemen (42) beaufschlagt wird. Das mittlere Band des Druckelements (32) liegt im ungespannten Zustand über der Ebene der beiden äußeren Bänder (34).

Das Druckelement (32) enthält an einem Ende ein Umlenkelement (38), während an seinem anderen Ende der Spannriemen (42) fest mit dem Material des Druckelements (32) verbunden ist. Weiterhin weist das Druckelement (32) einen Schriftzug auf, der nur bei korrekter Anordnung des Druckelements (32) auf der Bandage (60) in richtiger Orientierung vom Anwender zu lesen ist.

Das Spannmittel (30) besitzt armseitig im Druckelement (32) ein Hakenband (46), das auf einem korrespondierenden Flausch auf der Bandage (60) (Grundelement (12)) befestigt werden kann.

Der Zugindikator (40) des Spannriemens (42) besitzt auf dem nichtelastischen, an den elastischen Bereich angrenzenden Bereich eine Referenzfigur (62) in Form eines gleichseitigen Dreiecks, während die Form der Indikatorfigur (64) auf dem elastischen Bereich ebenfalls ein Dreieck (nicht gleichseitig im ungedehnten Zustand) ist.
Das Spannmittel (30) besitzt eine Breite von ca. 4 cm, das Druckelement (32) von ca. 6 cm, wobei die Bänder (34) eine Breite von ca. 1,5 cm und die Zwischenräume (36) von ca.
0,6 cm aufweisen, und wobei die Länge des Druckelements (32) ca. 11 cm beträgt.

### Beispiel 3:

In einer alternativen Ausführungsform weisen die Pelotten (20) der Bandage (60) aus Beispiel 2 zwei erhabene Bereiche (22) auf, die quer zum Muskel verlaufen.

### Beispiel 4:

Eine alternative Ausführungsform entspricht den Bandagen gemäß den Beispielen 1 - 3, jedoch ist die Bandage (60) nicht zu einem Schlauch vernäht, sondern integral in einem Stück gewebt. Die Abschlüsse zu Ober- und Unterarm sind dabei aus einem anderen Material oder in einer anderen Art gewebt als der Bandagenkörper.

### Beispiel 5:

Eine weitere alternative Ausführungsform des orthopädischen Hilfsmittels entspricht wiederum im wesentlichen den in den vorhergehenden Beispielen beschriebenen Bandagen, jedoch weist das Grundelement (12) zwei mögliche Befestigungsstellen für das zweite Befestigungsmittel (46) des Spannmittels (30) auf, so daß das Druckelement (32) des Spannmittels (30) so angeordnet werden kann, daß es wahlweise auf diejenige Pelotte einwirken kann, die die Muskeln und Sehnen zum inneren oder äußeren Epicondylus beaufschlagt.

### Beispiel 6:

In einer noch weiteren alternativen Ausführungsform der vorhergenannten erfindungsgemäßen orthopädischen Hilfsmittel (10) in Form einer Bandage (60) ist das Druckelement (32) so ausgestaltet, daß es beide Pelotten (20) überfaßt.

### Beispiel 7:

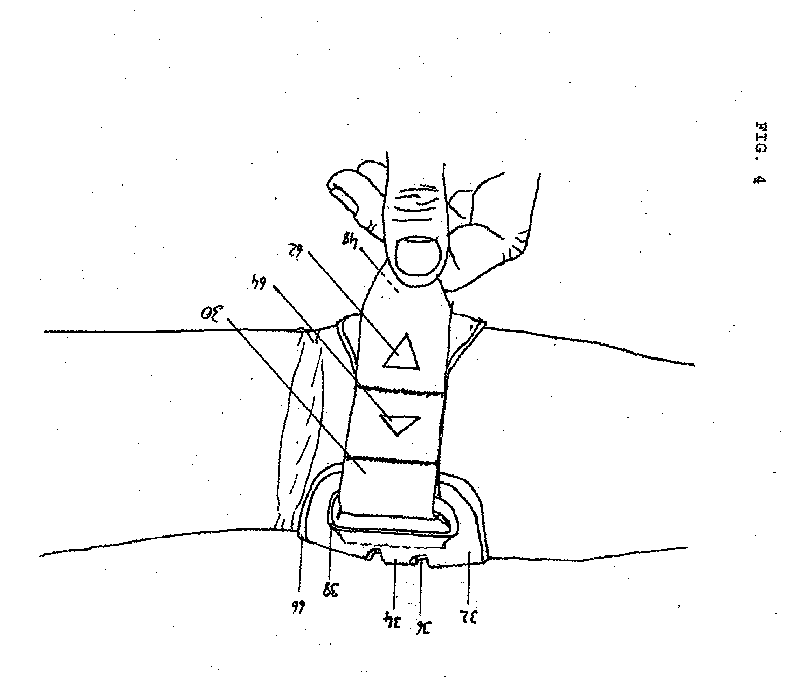

In einer anderen Ausführungsform der vorliegenden Erfindung ist das orthopädische Hilfsmittel (10) eine Epicondylitisspange, wie in Figur 4 dargestellt, wobei der Spannriemen (42) im wesentlichen analog zu den vorher beschriebenen Beispielen aufgebaut ist, während das Druckelement (32) als Grundkörper der Spange fungiert. Der Kunststoff des Druckelements (32) weist dabei eine höhere Flexibität auf als der Kunststoff bei Verwendung mit einer Bandage (60), um den Tragekomfort zu verbessern. Weiterhin ist die Länge des Druckelements (32) mindestens so groß, daß sie den halben Unterarm umfaßt.
Die Innenseite des Druckelements (32) ist mit einem dünnen Polster (66) aus einem geeigneten Material wie beispielsweise Kork, Filz oder einem Schaumstoff versehen und vollständig oder auch nur teilweise mit einem Flausch (50) bedeckt, der zum einen den Tragekomfort erhöht und zum anderen die Befestigung einer oder mehrerer Pelotten (20) an den entsprechenden Stellen ermöglicht. Die Pelotten (20) weisen wie auch zuvor erhabene Bereiche (22) auf, auf denen ein Hakenband zur Befestigung am Flausch im Druckelement angeordnet ist. Die Anordnung der Pelotten (20) auf der armseitigen (inneren) Oberfläche (14) der Bandage (60) erfolgt derart, daß die erhabenen Bereiche (22) auf den Bändern (34) des Druckelements (32) zu liegen kommen. Die Position der Pelotten (20) kann farblich oder mit Umrissen im Druckelement (32) markiert sein, um dem Anwender die korrekte Anordnung zu erleichtern.
Ferner sind die Pelotten (20) mit einem hautverträglichen Überzug versehen, wie z.B. einem Frottee-Stoff, der in der Lage ist, Schweiß aufzusaugen und ebenfalls den Tragekomfort zu erhöhen.

### Beispiel 8:

Eine weitere Ausführungsform analog zu Beispiel 7 betrifft ebenfalls eine Epicondylitisspange gemäß der vorliegenden Erfindung, wobei die Pelotten (20) aber direkt auf der armseitigen (inneren) Oberfläche (14) des Druckelements (32) durch Verkleben angeordnet sind. Sowohl das Druckelement (32) als auch die Pelotten (20) sind mit einem hautfreundlichen Stoff überzogen.

### Bezugszeichenliste

- 10: Orthopädisches Hilfsmittel
- 12: Grundelement
- 14: innere Oberfläche
- 16: äußere Oberfläche
- 18: Druckentlastungsbereich
- 20: Pelotte
- 22: erhabener Bereich
- 24: Oberarmabschluß
- 26: Unterarmabschluß
- 30: Spannmittel
- 32: Druckelement
- 34: Bänder
- 36: Zwischenräume
- 38: Umlenkelement
- 40: Zugindikator
- 41: flexibler Bereich
- 42: Spannriemen
- 44: Klettsystem / erstes Befestigungsmittel
- 46: zweites Befestigungsmittel
- 48: Hakenbandabschnitt
- 50: Flausch
- 60: Bandage
- 62: Referenzfigur
- 64: Indikatorfigur
- 66: Polster
- 70: Extensoren
- 72: Flexoren

## Patentansprüche

1. Orthopädisches Hilfsmittel (10), insbesondere zur Anwendung bei Epicondylitis, umfassend ein Grundelement (12) mit einer inneren, körperseitigen und einer äußeren Oberfläche (14,16) und mit mindestens einer Pelotte (20), und ein zumindest in einem Abschnitt elastisches Spannmittel (30) zur Druckbeaufschlagung zumindest eines Bereichs der Pelotte (20), **dadurch gekennzeichnet, daß** das Spannmittel (30) ein Druckelement (32) und einen dynamischen Zugindikator (40) zur Einstellung des vom Druckelement (32) ausgeübten Drucks auf die Pelotte (20) aufweist.

2. Orthopädisches Hilfsmittel (10) nach Anspruch 1, **dadurch gekennzeichnet, daß** das Spannmittel (30) ein Druckelement (32), ein Umlenkelement (38), einen zumindest in einem Abschnitt elastischen Spannriemen (42) mit einem Zugindikator (40) und ein erstes Befestigungsmittel (44) zur Fixierung des Spannriemens (42) unter Spannung, insbesondere ein Klettelement, umfaßt.

3. Orthopädisches Hilfsmittel (10) nach Anspruch 1 oder 2, **dadurch gekennzeichnet, daß** das Druckelement (32) des Spannmittels (30) ein mehrbändiges Element mit N-Bändern (34) und N-1 freien Zwischenräumen (36) zwischen den Bändern (34) ist, wobei N von 2-8, bevorzugt von 3-5, und besonders bevorzugt 3 ist, wobei die Breite der einzelnen Bänder (34) und der einzelnen Zwischenräume (36) unabhängig voneinander frei wählbar ist.

4. Orthopädisches Hilfsmittel (10) nach Anspruch 1 bis 3, **dadurch gekennzeichnet, daß** der Spannriemen (42) des Spannmittels (30) zumindest einen dehnbaren Abschnitt umfaßt; und wobei der Spannriemen (42) mit dem Druckelement (32) fest an einem Ende verbunden ist; und
wobei das freie Ende des Spannriemens (42) stufenlos verstellbar mit dem ersten Befestigungsmittel (44) auf dem dem Spannriemen (42) selbst oder dem Grundelement (12) zu befestigen ist.

5. Orthopädisches Hilfsmittel (10) nach einem der vorhergehenden Ansprüche, **dadurch gekennzeichnet, daß** das Druckelement (32) des Spannmittels (30) nur einbändig ist.

6. Orthopädisches Hilfsmittel (10) nach einem der vorhergehenden Ansprüche, **dadurch gekennzeichnet, daß** das Spannmittel (30) über ein zweites Befestigungsmittel (46) abnehmbar mit dem Grundelement (12) verbunden ist, wobei das zweite Befestigungsmittel (46) vorzugsweise ein Klettelement bestehend aus Flausch und Hakenband ist und vorzugsweise der Flausch auf dem Grundelement (12) und das Hakenband auf dem Spannmittel (30) angeordnet ist.

7. Orthopädisches Hilfsmittel (10) nach einem der vorhergehenden Ansprüche, **dadurch gekennzeichnet, daß** das Spannmittel (30) integral in einem Stück geformt ist und Spannriemen (42) und Druckelement (32) an einem Ende fest miteinander verbunden sind, inbesondere, das der Spannriemen (42) in das Druckelement (32) eingegossen ist.

8. Orthopädisches Hilfsmittel (10) nach einem der vorhergehenden Ansprüche, **dadurch gekennzeichnet, daß** das Druckelement (32) aus einem Material hergestellt ist, ausgewählt aus der Gruppe, die dehnungselastische und nicht dehnungselastische Gewebe und Kunststoffe, flexible biegungselastische Materialien und starre, nicht biegungselastische Materialien, oder Kombinationen der zuvor genannten Materialien umfaßt, wobei Nylon, Poylethylenterephthalat oder thermoplastisches Polyurethan bevorzugt sind.

9. Orthopädisches Hilfsmittel (10) nach einem der vorhergehenden Ansprüche, **dadurch gekennzeichnet, daß** die Breite der Bänder (34) des Druckelements (32) zwischen 0,2 cm und 3,0 cm, bevorzugt zwischen 0,5 cm und 2,0 cm, weiter bevorzugt zwischen 1,0 cm und 1,7 cm, und besonders bevorzugt 1,5 cm beträgt, und wobei die Breite der Zwischenräume (36) zwischen 0,2 cm und 3 cm, bevorzugt zwischen 0,5 cm und 2 cm, weiter bevorzugt zwischen 0,7 cm und 1,5 cm, und besonders bevorzugt 1,0 cm beträgt, wobei die Breite der einzelnen Bänder (34) und der einzelnen Zwischenräume (36) unabhängig voneinander frei wählbar ist.

10. Orthopädisches Hilfsmittel (10) nach einem der vorhergehenden Ansprüche, **dadurch gekennzeichnet, daß** das die Breite des Druckelements (32) im Verhältnis zur Breite der Pelotte frei gewählt werden kann.

11. Orthopädisches Hilfsmittel (10) nach einem der vorhergehenden Ansprüche, **dadurch gekennzeichnet, daß** die Position des mehrbändigen Abschnitts des Druckelements (32) auf der Pelotte fixiert wird, **dadurch** daß die äußeren Bänder (34) des Druckelements (32) unmittelbar an den Rändern der Pelotte verlaufen und/oder daß ein inneres Band in einer Vertiefung der Pelotte verläuft, wobei bevorzugt die fixierenden Bänder (34) tiefer im Druckelement (32) angeordnet sind als die pressenden Bänder (34).

12. Orthopädisches Hilfsmittel (10) nach einem der vorhergehenden Ansprüche, **dadurch gekennzeichnet, daß** die Pelotten (20) abnehmbar auf der körperseitigen Oberfläche (14) des Grundelements angeordnet sind.

13. Orthopädisches Hilfsmittel (10) nach einem der vorhergehenden Ansprüche, **dadurch gekennzeichnet, daß** die Pelotten (20) aus einem Material hergestellt sind, ausgewählt aus der Gruppe, die weichelastisch nicht kompressible Materialien, weichelastisch kompressiblen Materialien und nicht verformbare Materialien oder Kombinationen der zuvor genannten Materialien umfaßt, wobei die Pelotten bevorzugt aus Syntheselatex hergestellt sind.

14. Orthopädisches Hilfsmittel (10) nach einem der vorhergehenden Ansprüche, **dadurch gekennzeichnet, daß** die Pelotten (20) auf der dem Muskel abgewandten Seite und/oder der dem Muskel zugewandten Seite erhabene Bereiche (22) aufweisen, die quer zum oder in Richtung des Muskels verlaufen oder frei orientierbar oder gekreuzt sind.

15. Orthopädisches Hilfsmittel (10) nach einem der vorhergehenden Ansprüche, **dadurch gekennzeichnet, daß** die einzelnen Bänder (34) des Druckelements (32) so geformt sind, daß sie auf erhabenen Bereiche (22) und/oder in vertieften Bereichen der Pelotte liegen, und daß die Bänder (34) des Druckelements (32) auf die unterliegenden Bereiche der Pelotte einen Druck ausüben,

16. Orthopädisches Hilfsmittel (10) nach einem der vorhergehenden Ansprüche, **dadurch gekennzeichnet, daß** das Grundelement (12) zwei Pelotten (20) enthält, wobei die Pelotten (20) vorzugsweise symmetrisch, besonders bevorzugt symmetrisch in Längsrichtung des Hilfsmittels angeordnet sind.

17. Orthopädisches Hilfsmittel (10) nach einem der vorhergehenden Ansprüche, **dadurch gekennzeichnet, daß** der Zugindikator (40) eine auf einem elastischen Abschnitt aufgebrachte oder eingearbeitete als Indikatorfigur (64) bezeichnete geometrische Figur umfaßt, die bei Zug auf dem Spannriemen (42) ihre Form derart verändert, daß sie mit einer Referenzfigur (62), die einer Dehung bei einem vorgegebenen, bevorzugten Zug entspricht, übereinstimmt.

18. Orthopädisches Hilfsmittel (10) nach einem der vorhergehenden Ansprüche, **dadurch gekennzeichnet, daß** die geometrische Figur des Zugindikators (40) ein Kreis/Ellipse, ein Quadrat/Rechteck und besonders bevorzugt ein gleichseitiges Dreieck/Dreieck im jeweils gedehnten/ungedehnten Zustand ist.

19. Orthopädisches Hilfsmittel (10) nach einem der vorhergehenden Ansprüche, **dadurch gekennzeichnet, daß** der Zugindikator (40) auch mindestens eine Referenzfigur (62) umfaßt.

20. Orthopädisches Hilfsmittel (10) nach einem der vorhergehenden Ansprüche, **dadurch gekennzeichnet, daß** das Orthopädisches Hilfsmittel (10) eine Bandage (60) zur Behandlung oder Vorbeugung von Epicondylitis ist.

21. Orthopädisches Hilfsmittel (10) nach Anspruch 20, **dadurch gekennzeichnet, daß** das Grundelement (12) eine Bandage (60) aus einem elastischem Material in einer das Gelenk nicht umfassenden Schlauchform mit mindestens einer beaufschlagenden Druckpelotte ist.

22. Orthopädisches Hilfsmittel (10) nach Anspruch 21 **dadurch gekennzeichnet, daß** das Grundelement (12) eine schlauchförmige Bandage (60) aus einem elastischem Material ist, die den Ellenbogen nicht umfaßt; wobei
symmetrisch angeordneten Pelotten (20) zur Druckausübung auf beide zu den Epicondylen führenden Muskelgruppen in das Grundelement (12) eingearbeitet sind; und
die fest eingearbeiteten Pelotten (20) aus einem weichelastischen Material hergestellt sind;
die Pelotten (20) auf der dem Körper abgewandten Seite zwei längliche, erhabene Strukturen aufweisen, die quer zur Muskulatur verlaufen;
die Bandage (60) in Richtung zur Hand einen weiter gewebter Abschluß und in Richtung zum Ellenbogen einen abgesetzten, weiter gewebten Abschluß mit einem Druckentlastungsbereich (18) aufweist;
die Bandage (60) ein Spannmittel (30) zur zusätzlichen Druckbeaufschlagung insbesondere auf die auf den äußeren Epicondylus wirkende Pelotte durch ein dreibändiges, nicht flexibles Druckelement (32) aus einem festen, nicht dehnbaren Kunststoff aufweist;
das Druckelement (32) ein Umlenkelement (38) enthält; und der Spannriemen (42) einen flexiblen Abschnitt und einen nicht flexiblen Abschnitt umfaßt und der Spannriemen (42) einen dynamischen Zugindikator (40) enthält.

23. Orthopädisches Hilfsmittel (10) nach Anspruch 20 **dadurch gekennzeichnet, daß** das Grundelement (12) eine schlauchförmige Bandage (60) aus einem elastischem Material ist, die den Ellenbogen umfaßt; wobei
symmetrisch angeordneten Pelotten (20) zur Druckausübung auf beide zu den Epicondylen führenden Muskelgruppen in das Grundelement (12) eingearbeitet sind; und
die fest eingearbeiteten Pelotten (20) aus einem weichelastischen Material hergestellt sind;
die Bandage (60) in Richtung zur Hand einen weiter gewebter Abschluß und in Richtung zum Ellenbogen einen abgesetzten, weiter gewebten Abschluß mit einem Druckentlastungsbereich (18) aufweist;
die Bandage (60) ein Spannmittel (30) zur zusätzlichen Druckbeaufschlagung insbesondere auf die auf den äußeren Epicondylus wirkende Pelotte durch ein dreibändiges nicht flexibles Druckelement (32) aus einem festen, nicht dehnbaren Kunststoff aufweist;
das Druckelement (32) ein Umlenkelement (38) enthält; und der Spannriemen (42) einen flexiblen Abschnitt und einen nicht flexiblen Abschnitt umfaßt und der Spannriemen (42) einen dynamischen Zugindikator (40) enthält.
, wobei Indikator und Referenzfigur Dreiecke sind.

24. Orthopädisches Hilfsmittel (10) nach einem der vorhergehenden Ansprüche, **dadurch gekennzeichnet, daß** das Spannmittel (30) abnehmbar über eine Klettverbindung mit dem Grundelement (12) verbunden ist, so daß die Bandage (60) sowohl für den linken als auch rechen Arm verwendet als auch ohne Gurt werden kann.

25. Orthopädisches Hilfsmittel (10) nach einem der Ansprüche 1-19, **dadurch gekennzeichnet, daß** das orthopädisches Hilfsmittel (10) eine Spange zur Behandlung oder Vorbeugung von Epicondylitis ist.

26. Orthopädisches Hilfsmittel (10) nach Anspruch 25, **dadurch gekennzeichnet, daß** das Grundelement (12) eine Spange aus einem festen elastischem Material mit mindestens einer beaufschlagenden Druckpelotte ist.

27. Orthopädisches Hilfsmittel (10) nach Anspruch 25 oder 26, **dadurch gekennzeichnet, daß** das Druckelement (32) integraler Bestandteil des Grundelements (12) der Spange ist.

28. Spannmittel (30) zur Druckbeaufschlagung einer Pelotte, mit Druckelement (32), Spannriemen (42), Zugindikator (40) in einem zugelastischem Abschnitt des Spannriemens (42), einem ersten Befestigungsmittel zur Fixierung des Spannriemens (42) unter Spannung, und einem zweiten Befestigungsmittel (46) zur lösbaren Befestigung des Spannriemens (42) auf einem Grundelement (12), ausgewählt aus der Gruppe, die Spangen und Bandagen zur Behandlung der Epicondylitis umfaßt.

## Claims

1. Orthopaedic remedy (10), especially for use in cases of epicondylitis, comprising a base element (12) with an inner, body-side surface and an outer surface (14, 16) and with at least one pad (20), and a tightening means (30), which is elastic in at least a section thereof, for pressurization of at least a region of the pad (20), **characterized in that** the tightening means (30) has a pressure element (32) and a dynamic tension indicator (40) for adjusting the pressure exerted on a pad (20) by the pressure element (32).

2. Orthopaedic remedy (10) according to claim 1, **characterized in that** the tightening means (30) comprises a pressure element (32), a diverting element (38), a tightening strap (42), being elastic in at least a section thereof, with a tension indicator (40), and a first fastening means (44) for fixating the tightening strap (42) under tension, particularly a hook-and-loop element.

3. Orthopaedic remedy (10) according to claim 1 or 2, **characterized in that** the pressure element (32) of the tightening means (30) is a multi-band element with N bands (34) and N-1 free interspaces (36) between the bands (34), with N being 2-8, preferably 3-5 and more preferably 3, and with the width of the individual bands (34) and of the individual interspaces (36) being selectable independently from one another.

4. Orthopaedic remedy (10) according to claims 1 to 3, **characterized in that** the tightening strap (42) of the tightening means (30) comprises at least one extendible section; and
wherein the tightening strap (42) at one end thereof is permanently connected with the pressure element (32); and wherein the free end of the tightening strap (42) is to be fastened, in a continuously adjustable manner, by means of the first fastening means (44), on the tightening strap (42) itself or on the base element (12).

5. Orthopaedic remedy (10) according to any one of the preceding claims, **characterized in that** the pressure element (32) of the tightening means (30) comprises only one band.

6. Orthopaedic remedy (10) according to any one of the preceding claims, **characterized in that** the tightening means (30) is detachably connected with the base element (12) via a second fastening means (46), wherein the second fastening means (46) preferably is a hook-and-loop element, consisting of fleece and hook tape, and wherein, preferably, the fleece is arranged on the base element (12) and the hook tape on the tightening means (30).

7. Orthopaedic remedy (10) according to any one of the preceding claims, **characterized in that** the tightening means (30) is formed integrally in one piece, and that the tightening strap (42) and the pressure element (32) are permanently connected with one another at one end thereof, more particularly, that the tightening strap (42) is cast-in in the pressure element (32).

8. Orthopaedic remedy (10) according to any one of the preceding claims, **characterized in that** the pressure element (32) is made of a material selected from the group which comprises expandable-elastic fabrics and plastics and fabrics and plastics that are not expandable-elastic, flexible flexural-elastic materials, and rigid, non-flexural-elastic materials, or combinations of the aforementioned materials, with nylon, polyethylene terephthalate or thermoplastic polyurethane being preferred.

9. Orthopaedic remedy (10) according to any one of the preceding claims, **characterized in that** the width of the bands (34) of the pressure element (32) is between 0.2 cm and 3.0 cm, preferably between 0.5 cm and 2.0 cm, more preferably between 1.0 cm and 1.7 cm, and most preferably 1.5 cm, and wherein the width of the interspaces (36) is between 0.2 cm and 3 cm, preferably between 0.5 cm and 2 cm, more preferably between 0.7 cm and 1.5 cm, and most preferably 1.0 cm, and wherein the width of the individual bands (34) and of the individual interspaces (36) can be selected independently from one another.

10. Orthopaedic remedy (10) according to any one of the preceding claims, **characterized in that** the width of the pressure element (32) relative to the width of the pad can be selected freely.

11. Orthopaedic remedy (10) according to any one of the preceding claims, **characterized in that** the position of the multi-band section of the pressure element (32) is fixated due to the outer bands (34) of the pressure element (32) running directly along the edges of the pad, and/or due to an inner band running in a recess in the pad, with the fixating bands (34) preferably being arranged deeper in the pressure element (32) than the pressurizing bands (34).

12. Orthopaedic remedy (10) according to any one of the preceding claims, **characterized in that** the pads are detachably arranged on the body-side surface (14) of the base element.

13. Orthopaedic remedy (10) according to any one of the preceding claims, **characterized in that** the pads (20) are made of a material selected from the group which comprises soft-elastic non-compressible materials, soft-elastic compressible materials, and non-deformable materials, or combinations of the aforementioned materials, said pads preferably being made of synthetic latex.

14. Orthopaedic remedy (10) according to any one of the preceding claims, **characterized in that** on the side facing away from the muscle and/or on the side facing towards the muscle the pads (20) have elevated regions (22) which run transversely to or in the direction of the muscle, or which are freely orientable, or crossed.

15. Orthopaedic remedy (10) according to any one of the preceding claims, **characterized in that** the individual bands (34) of the pressure element (32) are formed such that they are located on elevated regions (22) and/or in recessed regions of the pad, and that the bands (34) of the pressure element (32) exert a pressure on the underlying regions of the pad.

16. Orthopaedic remedy (10) according to any one of the preceding claims, **characterized in that** the base element (12) contains two pads (20), wherein said pads (20) are preferably arranged symmetrically, more preferably symmetrically in the longitudinal direction of the remedy.

17. Orthopaedic remedy (10) according to any one of the preceding claims, **characterized in that** the tension indicator (40) comprises a geometrical shape applied to or incorporated in an elastic section and referred to as indicator shape (64) which when the tightening strap (42) is tensioned changes its shape such that it coincides with a reference shape (62) that corresponds to an extension at a prespecified, preferred tension.

18. Orthopaedic remedy (10) according to any one of the preceding claims, **characterized in that** the geometrical shape of the tension indicator (40) is a circle/ellipse, a square/rectangle, and especially preferably an isosceles triangle/triangle, in the elongated/non-elongated state, respectively.

19. Orthopaedic remedy (10) according to any one of the preceding claims, **characterized in that** the tension indicator (40) also comprises at least one reference shape (62).

20. Orthopaedic remedy (10) according to any one of the preceding claims, **characterized in that** the orthopaedic remedy (10) is a bandage (60) for the treatment or for the prophylaxis of epicondylitis.

21. Orthopaedic remedy (10) according to claim 20, **characterized in that** the base element (12) is a bandage (60) made of an elastic material which is of a tubular shape not encompassing the joint and which has at least one pressurizing pressure pad.

22. Orthopaedic remedy (10) according to claim 21, **characterized in that** the base element (12) is a tubular bandage (60) of an elastic material which does not encompass the elbow; wherein
symmetrically arranged pads (20) are incorporated in the base element (12) for applying a pressure against both muscle groups leading to the epicondyles; and
the permanently incorporated pads (20) are made of a soft-elastic material;
the pads (20) have two elongate, elevated structures on the side facing away from the body, which structures extend transversely to the musculature;
the bandage (60) comprises, in the direction towards the hand, a more loosely woven border and, in the direction towards the elbow, a trimmed, more loosely woven border with a pressure release region (18);
the bandage (60) has a tightening means (30) for additional pressure application, especially against that pad which acts on the external epicondyle, by means of a three-band, nonflexible pressure element (32) of a firm, non-flexible plastic;
the pressure element (32) contains a diverting element (38); and
the tightening strap (42) comprises a flexible section and a non-flexible section, and the tightening strap (42) contains a dynamic tension indicator (40).

23. Orthopaedic remedy (10) according to claim 20, **characterized in that** the base element (12) is a tubular bandage (60) of an elastic material which encompasses the elbow; wherein
symmetrically arranged pads (20) are incorporated in the base element (12) for applying a pressure against both muscle groups leading to the epicondyles; and
the permanently incorporated pads (20) are made of a soft-elastic material;
the bandage (60) comprises, in the direction towards the hand, a more loosely woven border and, in the direction towards the elbow, a trimmed, more loosely woven border with a pressure release region (18);
the bandage (60) has a tightening means (30) for additional pressure application, especially against that pad which acts on the external epicondyle, by means of a three-band, nonflexible pressure element (32) of a firm, non-flexible plastic;
the pressure element (32) contains a diverting element (38); and
the tightening strap (42) comprises a flexible section and a non-flexible section, and the tightening strap (42) contains a dynamic tension indicator (40),
with the indicator and the reference shape being triangles.

24. Orthopaedic remedy (10) according to any one of the preceding claims, **characterised in that** the tightening element (30) is detachably connected with the base element (12) by means of a hook-and-loop connection, so that the bandage (60) can be used both for the left and the right arm, as well as without a belt.

25. Orthopaedic remedy (10) according to any one of claims 1-19, **characterised in that** the orthopaedic remedy (10) is a clasp for the treatment or prophylaxis of epicondylitis.

26. Orthopaedic remedy (10) according to claim 25, **characterised in that** the base element (12) is a clasp of a firm elastic material with at least one pressurizing pressure pad.

27. Orthopaedic remedy (10) according to claim 25 or 26, **characterised in that** the pressure element (32) is an integral part of the base element (12) of the clasp.

28. Tightening means (30) for applying a pressure against a pad, having a pressure element (32), a tightening strap (42), a tension indicator (40) in a tensile-elastic section of the tightening strap (42), a first fastening means for fixating the tightening strap (42) under tension, and a second fastening means (46) for releasably attaching the tightening strap (42) on a base element (12), selected from the group comprising clasps and bandages for treating epicondylitis.

## Revendications

1. Remède orthopédique (10), en particulier en vue d'une application dans le cas de l'épicondylite, comprenant un élément de base (12) avec une surface intérieure du côté corps et une surface extérieure (14, 16) et avec au moins une pelote (20), et un moyen de serrage (30) au moins élastique dans une section en vue d'une mise sous pression d'au moins une région de la pelote (20), **caractérisé en ce que** le moyen de serrage (30) présente un élément de pression (32) et un indicateur de traction dynamique (40) pour ajuster la pression exercée par l'élément de pression (32) sur la pelote (20).

2. Remède orthopédique (10) selon la revendication 1, **caractérisé en ce que** le moyen de serrage (30) comprend un élément de pression (32), un élément déflecteur (38), une sangle de serrage (42) au moins élastique dans une section avec un indicateur de traction (40) et un premier moyen de fixation (44) pour fixer la sangle de serrage (42) sous contrainte, en particulier un élément velcro.

3. Remède orthopédique (10) selon la revendication 1 ou 2, **caractérisé en ce que** l'élément de pression (32) du moyen de serrage (30) est un élément à plusieurs bandes avec N bandes (34) et N-1 interstices libres (36) entre les bandes (34), N valant de 2 à 8, de préférence de 3 à 5, et de manière particulièrement préférée valant 3, la largeur des bandes individuelles (34) et des interstices individuels (36) pouvant être choisie librement indépendamment l'une de l'autre.

4. Remède orthopédique (10) selon la revendication 1 à 3, **caractérisé en ce que** la sangle de serrage (42) du moyen de serrage (30) comprend au moins une section extensible ; et dans lequel la sangle de serrage (42) est solidarisée à l'élément de pression (32) à une extrémité ; et
dans lequel l'extrémité libre de la sangle de serrage (42) est ajustable en continu avec le premier moyen de fixation (44) sur la sangle de serrage (42) elle-même, ou bien doit être fixée à l'élément de base (12).

5. Remède orthopédique (10) selon l'une quelconque des revendications précédentes, **caractérisé en ce que** l'élément de pression (32) du moyen de serrage (30) est seulement à une bande.

6. Remède orthopédique (10) selon l'une quelconque des revendications précédentes, **caractérisé en ce que** le moyen de serrage (30) est raccordé de manière amovible à l'élément de base (12) par l'intermédiaire d'un deuxième moyen de fixation (46), dans lequel le deuxième moyen de fixation (46) de préférence un élément velcro constitué de velours, constitue une bande à crochets, et de préférence le velours est disposé sur l'élément de base (12) et la bande à crochets sur le moyen de serrage (30).

7. Remède orthopédique (10) selon l'une quelconque des revendications précédentes, **caractérisé en ce que** le moyen de serrage (30) est formé entièrement en une pièce, et une sangle de serrage (42) et un élément de pression (32) sont solidarisés l'un à l'autre au niveau d'une extrémité, en particulier **en ce que** la sangle de serrage (42) est coulée dans l'élément de pression (32).

8. Remède orthopédique (10) selon l'une quelconque des revendications précédentes, **caractérisé en ce que** l'élément de pression (32) est fabriqué dans un matériau, choisi dans le groupe qui comprend les tissus et matières plastiques élastiques en extension et non élastiques en extension, les matériaux souples élastiques en flexion et les matériaux solides non élastiques en flexion, ou les combinaisons des matériaux précités, le nylon, le poly(téréphtalate d'éthylène) ou le polyuréthane thermoplastique étant préférés.

9. Remède orthopédique (10) selon l'une quelconque des revendications précédentes, **caractérisé en ce que** la largeur des bandes (34) de l'élément de pression (32) est comprise entre 0,2 cm et 3,0 cm, de préférence comprise entre 0,5 cm et 2,0 cm, de manière particulièrement préférée comprise entre 1,0 cm et 1,7 cm, et de manière tout particulièrement préférée de 1,5 cm, et la largeur des interstices (36) étant comprise entre 0,2 cm et 3 cm, de préférence entre 0,5 cm et 2 cm, de manière particulièrement préférée entre 0,7 cm et 1,5 cm, et de manière tout particulièrement préférée de 1,0 cm, la largeur des bandes individuelles (34) et celle des interstices individuels (36) pouvant être choisies librement indépendamment l'une de l'autre.

10. Remède orthopédique (10) selon l'une quelconque des revendications précédentes, **caractérisé en ce que** la largeur de l'élément de pression (32) par rapport à la largeur de la pelote, peut être choisie librement.

11. Remède orthopédique (10) selon l'une quelconque des revendications précédentes, **caractérisé en ce que** la position de la section à plusieurs bandes de l'élément de pression (32) est fixée sur la pelote, du fait que les bandes extérieures (34) de l'élément de pression (32) se prolongent directement sur les bords de la pelote et/ou qu'une bande intérieure se prolonge dans un creux de la pelote, les bandes fixantes (34) étant disposées plus profondément dans l'élément de pression (32) que les bandes pressantes (34).

12. Remède orthopédique (10) selon l'une quelconque des revendications précédentes, **caractérisé en ce que** les pelotes (20) sont disposées de façon amovible sur la surface (14) du côté corps de l'élément de base.

13. Remède orthopédique (10) selon l'une quelconque des revendications précédentes, **caractérisé en ce que** les pelotes (20) sont fabriquées en un matériau choisi dans le groupe qui comprend les matériaux élastiquement mous non compressibles, les matériaux élastiquement mous compressibles, et les matériaux non déformables ou les combinaisons des matériaux précités, les pelotes étant de préférence fabriquées en latex de synthèse.

14. Remède orthopédique (10) selon l'une quelconque des revendications précédentes, **caractérisé en ce que** les pelotes (20) présentent des régions en relief (22) sur le côté faisant dos au muscle et/ou sur le côté tourné vers le muscle, lesquelles se prolongent transversalement au muscle ou dans la direction de celui-ci, ou bien peuvent être orientées librement, ou sont croisées.

15. Remède orthopédique (10) selon l'une quelconque des revendications précédentes, **caractérisé en ce que** les bandes individuelles (34) de l'élément de pression (32) sont formées de façon à se trouver sur des régions en relief (22) et/ou dans des régions en creux de la pelote, et que les bandes (34) de l'élément de pression (32) exercent une pression sur les régions sous-jacentes de la pelote.

16. Remède orthopédique (10) selon l'une quelconque des revendications précédentes, **caractérisé en ce que** l'élément de base (12) contient deux pelotes (20), les pelotes (20) étant disposées de préférence symétriquement, de manière particulièrement préférée symétriquement dans la direction longitudinale du remède.

17. Remède orthopédique (10) selon l'une quelconque des revendications précédentes, **caractérisé en ce que** l'indicateur de traction (40) comprend une figure géométrique appliquée sur une section élastique ou incorporée en tant que figure indicatrice (64), qui lors d'une traction sur la sangle de serrage (42) modifie sa forme de sorte qu'elle coïncide avec une figure de référence (62), qui correspond à un allongement pour une traction préférée prédéterminée.

18. Remède orthopédique (10) selon l'une quelconque des revendications précédentes, **caractérisé en ce que** la figure géométrique de l'indicateur de traction (40) constitue une figure cercle/ellipse, carré/rectangle, et de manière particulièrement préférée triangle équilatéral/triangle, respectivement à l'état allongé/non allongé.

19. Remède orthopédique (10) selon l'une quelconque des revendications précédentes, **caractérisé en ce que** l'indicateur de traction (40) comprend également au moins une figure de référence (62).

20. Remède orthopédique (10) selon l'une quelconque des revendications précédentes, **caractérisé en ce que** le remède orthopédique (10) est un bandage (60) pour traiter et prévenir l'épicondylite.

21. Remède orthopédique (10) selon la revendication 20, **caractérisé en ce que** l'élément de base (12) est un bandage (60) constitué d'un matériau élastique, en forme de tuyau ne comprenant pas l'articulation, avec au moins une pelote de mise sous pression.

22. Remède orthopédique (10) selon la revendication 21, **caractérisé en ce que** l'élément de base (12) est un bandage (60) en forme de tuyau constitué d'un matériau élastique, qui ne comprend pas le coude ; des pelotes disposées symétriquement (20) pour exercer une pression sur les deux groupes musculaires conduisant aux épicondyles sont incorporées dans l'élément de base (12) ; et
les pelotes solidement incorporées (20) sont fabriquées en un matériau mou élastique,
les pelotes (20) présentent sur le côté faisant dos au corps deux structures en relief longitudinales qui se prolongent transversalement à la musculature ;
le bandage (60) dans la direction de la main présente une bordure encore tissée, et dans la direction du coude une bordure déposée encore tissée avec une région de détente de pression (18) ;
le bandage (60) présente un moyen de serrage (30) pour une mise sous pression supplémentaire en particulier de la pelote agissant sur l'épicondyle extérieure à l'aide d'un élément de pression à trois bandes, non flexible (32) constitué d'une matière plastique solide non extensible ;
l'élément de pression (32) contient un élément déflecteur (38) et la sangle de serrage (42) comprend une section souple et une section non souple, et la sangle de serrage (42) contient un indicateur de traction dynamique (40).

23. Remède orthopédique (10) selon la revendication 20, **caractérisé en ce que** l'élément de base (12) est un bandage en forme de tuyau (60) constitué d'un matériau élastique, qui comprend le coude ; des pelotes (20) disposées symétriquement pour exercer une pression sur les deux groupes musculaires conduisant aux épicondyles sont incorporés dans l'élément de base (12) ; et
les pelotes solidement incorporées (20) sont fabriquées en un matériau mou élastique ;
le bandage (60) dans la direction de la main présente une bordure encore tissée et dans la direction du coude une bordure déposée encore tissée avec une région de détente de pression (18) ;
le bandage (60) présente un moyen de serrage (30) pour une mise sous pression supplémentaire, en particulier de la pelote agissant sur l'épicondyle extérieure à l'aide d'un élément de pression à trois bandes, non flexible (32) constitué d'une matière plastique solide non extensible ;
l'élément de pression (32) contient un élément déflecteur (38) ; et
la sangle de serrage (42) comprend une section souple et une section non souple et la sangle de serrage (42) contient un indicateur de traction dynamique (40),
l'indicateur et la figure de référence étant des triangles.

24. Remède orthopédique (10) selon l'une quelconque des revendications précédentes, **caractérisé en ce que** le moyen de serrage (30) est raccordé de façon amovible à l'élément de base (12) par l'intermédiaire d'un raccord velcro, de sorte que le bandage (60) peut être employé aussi bien pour le bras gauche que pour le bras droit, ainsi qu'également sans ceinture.

25. Remède orthopédique (10) selon l'une quelconque des revendications 1 à 19, **caractérisé en ce que** le remède orthopédique (10) est une boucle pour traiter ou prévenir l'épicondylite.

26. Remède orthopédique (10) selon la revendication 25, **caractérisé en ce que** l'élément de base (12) est une boucle constituée d'un matériau solide élastique ayant au moins une pelote de mise sous pression.

27. Remède orthopédique (10) selon la revendication 25 ou 26, **caractérisé en ce que** l'élément de pression (32) fait partie intégrante de l'élément de base (12) de la boucle.

28. Moyen de serrage (30) pour la mise sous pression d'une pelote, avec un élément de pression (32), une sangle de serrage (42), un indicateur de traction (40) dans une section de la sangle de serrage (42), élastique en traction, un premier moyen de fixation pour fixer la sangle de serrage (42) sous contrainte et un deuxième moyen de fixation (46) pour fixer de manière amovible la sangle de serrage (42) sur un élément de base (12), choisis dans le groupe qui comprend les boucles et bandages pour traiter l'épicondylite.
